# EUROPEAN PATENT SPECIFICATION

(11) **EP 1 644 059 B1**
(45) Date of publication and mention of the grant of the patent: **22.01.2014**
(21) Application number: 04755719.4
(22) Date of filing: 18.06.2004
(51) Int. Cl.: A61M 1/00, A61M 3/02

(54) **PRECISION FLUID DELIVERY SYSTEM AND METHOD FOR SURGICAL PROCEDURES**
PRÄZISIONSFLUIDABGABESYSTEM UND VERFAHREN FÜR CHIRURGISCHE EINGRIFFE
SYSTEME DE DISTRIBUTION DE FLUIDE DE PRECISION ET PROCEDE DESTINE A DES PROCEDURES CHIRURGICALES

(30) Priority: 20.06.2003 US 600118
(43) Date of publication of application: 12.04.2006
(73) Proprietor: Sound Surgical Technologies, LLC, Louisville, CO 80027 (US)
(72) Inventor: CIMINO, William, W., Louisville, CO 80027 (US)
(74) Representative: Vossius & Partner
(86) International application number: PCT/US2004/019719
(87) International publication number: WO 2004/112579

(56) References cited:
- EP-A2- 0 446 897
- EP-A2- 0 978 361
- WO-A1-97/16220
- US-A- 6 024 720
- US-B1- 6 350 276

## Description

### I. FIELD OF THE INVENTION

This invention relates generally to fluid delivery systems, and more particularly, to precision fluid delivery systems for use in cosmetic surgical procedures where rapid and precise delivery of relatively large volumes of sterile fluid is required.

### II. BACKGROUND OF THE INVENTION

In general surgical procedures frequently involve the loss of significant quantities of blood and other bodily fluids, which must be replaced for recovery and healing. Crude procedures or "rules of thumb" have been developed for estimating fluid loss and the amount and nature of fluids prescribed for replacement. Generally, these fluids, such as blood or plasma, are delivered intravenously to the patient during the operation and post operative care. The fluid is delivered by gravity from an elevated reservoir through a tube and implanted needle. These "drip" systems are deliberately designed to deliver the fluid slowly to the patient. Markings indicating volume corresponding to the level of fluid in the reservoir (e.g., a transparent or translucent, plastic "bag") are used to estimate the amount of liquid administered to the patient.

Cosmetic surgery often requires the administration of fluids into a patient for other reasons. As used herein "cosmetic surgery" refers to invasive procedures that are used, at least in part to directly improve the appearance of a patient. For example, lipoplasty involves the removal of excess fatty tissue to improve the appearance and often the health of the patient. During lipoplasty fatty tissue is preferably separated from the remaining tissue with the assistance of an ultrasonic surgical instrument, and the separated fatty tissue is periodically removed from the patient by aspiration. A saline "wetting" solution is injected into the patient's fatty tissues at various times during the procedure both to assist in flushing the separated tissue from adjacent tissue and to mitigate damage to adjacent tissue from operation of the ultrasonic device. The wetting solution may contain drug additives such as epinephrine for vasoconstriction or lidocaine for suppression of pain.

Precision is important; the surgeon needs to know exactly how much fluid was inserted and exactly how much was taken out to properly perform any lipoplasty procedure. Precise volume information also helps to ensure symmetry and proportion where surgery is being conducted on adjacent body parts. To avoid prolonging the operation and to provide the desired final result, it is also highly desirable that the surgeon be able to deliver the fluid rapidly and in the precise amount to the body area being "sculpted."

Similarly, cosmetic surgery frequently involves the insertion of devices, e.g., plastic breast implants or temporary sizers, that are filled with sterile fluid to the desired amount. Again, it is highly desirable that the surgeon be able to insert the fluid rapidly in the precise volume. Standard procedures currently employed to fill implants and sizers use a syringe repeatedly to inject and monitor the appropriate amount of fluid. Such a process is inordinately slow

Currently available fluid delivery systems are inadequate to achieve these objectives. "Drip" methods typically employed to administer drugs or deliver other fluids are too slow and imprecise for the foregoing types of surgery. Further, they do not develop sufficient pressure to infuse the fatty tissues with the wetting solution. Surgical operations of the type mentioned previously would be unduly prolonged and the patient subjected to undue trauma if "drip" systems were employed to deliver the fluids. Drip systems also do not provide precise information about the amount of fluid delivered to the patient. To provide greater precision, "in-line" systems have been designed in which a paddle wheel is imposed in the tubing conveying the fluid. An infrared device "counts" the rotations of the paddle wheel caused by passage of the fluid, and a computer integrates that information over time to extrapolate the volume of liquid dispensed. These systems lack the required accuracy. They also place a mechanical measurement device, i.e., the paddle wheel, in direct contact with the sterile fluid thereby presenting the prospect of undesirable contamination. Attempts have also been made to use an encoder-based counter to count revolutions of the motor that drives the heads of a peristaltic pump that delivers the fluid. A computer integrates the total revolutions and the estimated volume ejected per revolution in an effort to calculate the total volume of fluid delivered. These systems also do not provide the requisite precision, since there is a significant variation in accuracy across the speed range of the pump.

Accordingly, there is a need for a fluid delivery system that will provide sterile fluids rapidly in precise volumes for use in medical procedures, especially those employed in cosmetic surgery.

In particular it is an object of the present invention to provide a fluid delivery system for use in a surgical environment that has a precision of +/- 1 ml over any volume from 10 ml to 5000 ml. In addition it is an object of the present invention to provide a fluid delivery system for use in a surgical environment that can rapidly deliver fluids at rates from 30 ml/min to 1000 ml/min. Such a system is particularly needed and suited for delivering wetting solution during lipoplasty and in the process of filling implanted or implantable breast implants.

### III. BRIEF DESCRIPTION OF THE INVENTION

It has now been found that liquids, such as sterile fluids can be rapidly and precisely delivered to a patient or for the filling of implantable devices using the direct measurement of fluid weight and a pump and tubing set. The performance and accuracy are enhanced by using a positive displacement pump and by using a tubing set of a non-distensible material.

It has been found that an acceptable system for delivering fluid in a surgical environment comprises: (1) a strain gauge sensor;
(2) a container of fluid connected to the strain-gauge sensor so that the strain-gauge sensor will measure the weight of the container of fluid and generate an electrical output proportional to the weight of the fluid and container from time-to-time; (3) a pump system for pumping fluid from the container and having adjustable speed control for delivery of fluids within the range of 30 ml/min to 1000 ml/min; (4) a sterile tubing set connected to the fluid source and passing through the pump system and for delivery of the fluid to the surgical environment (i.e., a patient or implantable device); (5) a processor for processing the electrical output from the strain gauge from time-to-time to determine the amount of fluid delivered to the surgical environment; and (6) a display for displaying the amount of fluid delivered by the surgical device. Preferably, the tubing set is designed to eliminate distension under the pressure generated by the pump system to improve the accuracy of the system and the information displayed.

Using this system and method, it is possible to obtain readings of +/- 1 ml over any required volume of interest while delivering fluid at a rate of 30 to 1000 ml./min.

### IV. BRIEF DESCRIPTION OF THE DRAWING

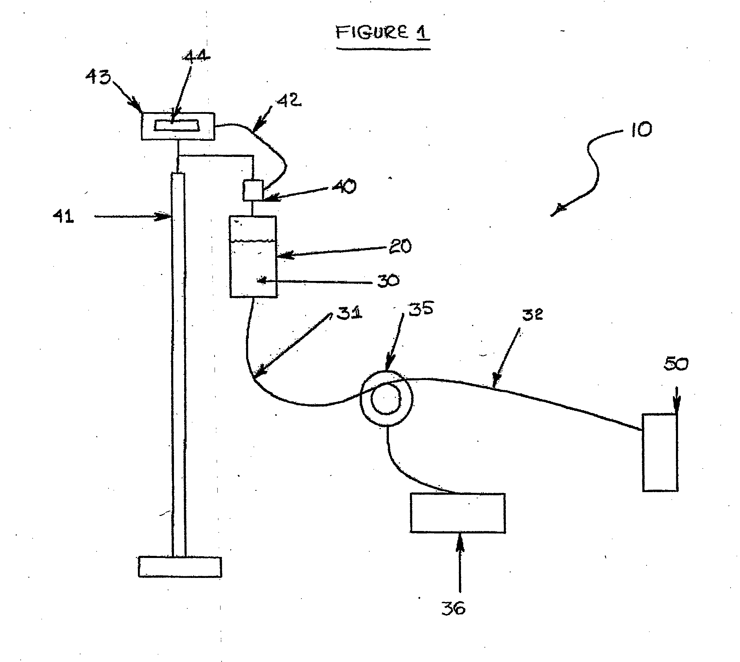

Figure 1 is a schematic representation of one embodiment of the present invention.

### V. DETAILED DESCRIPTION OF THE INVENTION AND THE PREFERRED EMBODIMENT

This invention is directed to the rapid delivery of relatively large volumes of fluid in a very precise manner in a surgical environment. Volumes of fluid of interest are generally greater than 10 ml and may range as high as 3000 to 5000 ml depending on the anatomical site. For precise delivery of wetting solution to tissues of a patient during the course of liopoplasty, volumes of interest may range from 100 ml to 5000 ml. Infusion rates may be from 100 ml/min to around 600 ml/min, although faster or slower rates may be used. For the rapid and accurate filling of saline breast implants and sizers, volumes of interest are typically from 100 to 500 ml.

It has now been discovered that a fluid delivery system can be provided capable of delivering fluid rapidly for surgical uses and having a measurement accuracy of approximately +/- 1 ml over required volumes of interest. Such a system uses the precise measurement of the weight of a fluid source over time, i.e., as fluid is delivered from the source. This is far superior to methods in which the volume of fluid delivered is implied from indicia of fluid flow (as previously referenced) and integrated over time to obtain the total volume of fluid moved. The system of the invention may also employ a fluid pumping device that positively displaces fluids from the fluid source to the patient. Finally the system of the invention may also employ a tubing set for delivery of the fluid from the fluid source to the patient that is of sufficient integrity that the tubing does not distend appreciably under the pressure generated by the fluid pumping device. While the tubing should be flexible enough that it is connected easily to the equipment, it should not distend or expand appreciably. Thus, the weight loss measured by the strain gauge reflects movement of the fluid into the patient or implanted device, rather than an increase in the volume of fluid in the tubing set due to expansion of the tubing.

In particular, it has been discovered that using a strain gauge to measure the weight (i.e., mass) of a bag of sterile fluid over time provides sufficient accuracy to measure the loss of fluid from the bag as required in surgical applications such as the cosmetic surgery procedures previously noted. Indeed, such a system has been able to obtain +/- 1 ml readings over any volume from 100 ml to 3000 ml. Strain gauges suitable for use in the present invention include devices with about a 10 pound limit and 5 volt excitation, such as the LC703-10, manufactured by Omega Engineering, Stamford, CT. The fluid container may be suspended from or otherwise connected to or supported by the strain-gauge so that accurate weight measurements are obtained. A simple hook system for suspending a bag of fluid is most adequate.

The strain-gauge provides an electronic signal proportional to the weight of the fluid source, i.e., fluid and container. A simple a strain gauge display may be employed to process the electronic signal output from the strain-gauge and, after calibration, display the current weight of the fluid source. A more complex strain gauge processor may be employed with appropriate memory capacity so that more detailed information can be displayed showing, for example, the amount of fluid delivered at various times or sequences as helpful to a surgeon performing a lipoplasty procedure in monitoring the administration of wetting solution and replacement of withdrawn fluids. In any event, suitable processing and display capability should be employed to translate the electronic signal from the strain gauge and display it as an accurate weight or volume. Such a device is the strain gauge meter model DI50-E-DR-PS1-IS01 manufactured by Texmate Inc., Vista, CA.. Preferably, the display has a reset button that will 'zero' the display when pressed.

In addition, it is highly desirable to employ a fluid pumping device that positively displaces fluids from the fluid source to the patient. For example, it has been found that a peristaltic pumping system, which is a positive displacement pump design, can be used to deliver the fluids rapidly and can be adjusted with regard to speed to deliver the fluids at different rates. In general, it is desirable that the pump be capable of providing fluids at the one or more rates within the range of about 30 ml/min to 1000 ml/min. For the convenience of the surgeon it is most desirable that the rate be completely variable within all or some portion of that range so that the fluid can be provided faster or slower depending upon the stage of the procedure in which the surgeon is engaged. At the very least, it is desirable that the surgeon should have at least several options to provide variability in flow rates. It is important to note that the rate of flow does not affect the accuracy of fluid flow measurement, since the fluid movement is not inferred from the activity of the pump, but from the loss of fluid and hence weight from the fluid source. Positive displacement pumps, preferably peristaltic pumps, are most efficient, result in accurate displacement of the fluid and are easily adjusted in speed. The use of such a pump ensures that the weight will be measured by the strain gauge simultaneously with the removal of fluid from the source and its delivery to the site where needed.

Finally, it is desirable that the system of the invention incorporates a tubing set for delivery of the fluid from the fluid source to the patient that is of sufficient integrity that the tubing set does not distend significantly under pump pressure. Tubing sets constructed of polyvinyl chloride, i.e., "PVC," with appropriate adapters on the ends and wall thicknesses transfer the fluids from the fluid source to the patient without excessive distension, thus guaranteeing that fluid pumped from the bag is actually delivered to the patient or implant. For example, a PVC tube with an inner diameter of 3/16^{th} inches and an outer diamater of 5/16^{th} inches will work well.

Figure 1 schematically depicts one specific embodiment of the present invention in which fluid delivery system 10 is used to provide wetting solution to patient 50 undergoing a lipoplasty procedure. A fluid source comprising container 20 and fluid 30 is suspended from strain gauge 40 which itself is suspended from the usual stand 41, commonly found in hospitals for suspending blood bags and similar fluid sources. In the same fashion, the container 20 of the present invention may also be a disposable plastic bag prepackaged to contain a specified amount of sterile wetting solution 30. In the embodiment depicted the disposable pre-packaged wetting solution is a 1000 ml plastic bag of sterile saline available from a large number of medical suppliers..

The fluid 30 is transported from container 20 to patient 50 using peristaltic pump 35. In the specific system illustrated this pump is a Watson Marlow pump design, model 313D2 available from Watson Marlow Bredel Inc., Wilmington, MA. The pump is connected via a tubing set 31 and 32 to the fluid source and the surgical instrument (not shown), respectively. The tubing set 32 is made of Tygon® (a registered trademark of the Norton Company of Worcester, Massachusetts) brand of polyvinyl chloride. Other brands of PVC tubing will work equally as well.

The pump 35 is connected to controller 36, which permits adjustment of the speed of the pump system. In this system the pump motor and controller are manufactured by Oriental Motor USA Corp., Los Angeles, CA, model AXU425. Thus, the surgeon can control the rate of flow of fluid from the supply 20 to the patient using the controller.

Finally, the strain gauge 20 is electronically connected via wire 42 to processing unit 43 and display 44.

The novel features of the invention are set forth in the appended claims and in light of this specification and drawing. It should be apparent to one skilled in the art that other alternatives are available to those specifically disclosed within this application and can be employed and within the scope of the appended claims.

## Claims

1. A system for delivering and accurately monitoring the delivery of a volume of interest of sterile fluid into a patient or an implantable device in a cosmetic surgery procedure, the system comprising:
a strain gauge sensor (40);
a container (20) of sterile fluid connected to the strain-gauge sensor so that the strain-gauge sensor will generate an electrical output proportional to the weight of the fluid and container from time-to-time;
a peristaltic pump (35) for pumping the volume of interest of the sterile fluid from the container into the patient or the implantable device, the peristaltic pump having an adjustable speed control to deliver the sterile fluid at one or more rates being within the range of 30 ml/min to 1000 ml/min, wherein the volume of interest ranges from 100 ml to 5000 ml;
a sterile tubing set (31, 32) connected to the container and the pump for delivery of the sterile fluid during the surgical procedure;
a processor (43) for processing the electrical output from the strain gauge from time-to-time to determine the volume of fluid delivered for the surgical procedure,
wherein the one or more rates do not affect the accuracy of fluid measurement and the fluid measurement is not inferred from the activity of the pump, but from a loss of fluid and hence weight from the container; and
a display (44) for displaying the amount of fluid delivered during the surgical procedure.

2. The system of claim 1 wherein the cosmetic surgery procedure is a member of the group consisting of lipoplasty and the filling of breast implants or sizers.

3. The system of claim 1 wherein the display includes a reset button that will 'zero' the display when pressed.

4. The system of claim 1 or 2, wherein the tubing set is made of polyvinyl chloride.

5. The system of claim 1 or 2, wherein the display shows the amount of fluid in either weight or volume.

## Patentansprüche

1. System zur Abgabe und präzisen Überwachung der Abgabe eines gewünschten Volumens eines sterilen Fluides in einen Patienten oder in eine implantierbare Vorrichtung während eines kosmetischen chirurgischen Eingriffs, wobei das System aufweist:
einen Spannungssensor (40);
einen Behälter (20) mit sterilem Fluid, der so mit dem Spannungssensor verbunden ist, dass der Spannungssensor in Zeitabständen ein elektrisches Ausgangssignal erzeugt, das proportional zum Gewicht des Fluides und Behälters ist;
eine Peristaltikpumpe (35) zum Pumpen des gewünschten Volumens des sterilen Fluides aus dem Behälter in den Patienten oder in die implantierbare Vorrichtung, wobei die Peristaltikpumpe eine einstellbare Geschwindigkeitssteuerung hat, um das sterile Fluid mit einer oder mehreren Raten im Bereich von 30ml/min bis 1000ml/min abzugeben, wobei das gewünschte Volumen zwischen 100ml und 5000ml beträgt;
eine mit dem Behälter und der Pumpe verbundene sterile Schlauchanordnung (31,32) zur Abgabe des sterilen Fluides während des chirurgischen Eingriffs;
einen Prozessor (43) zum Verarbeiten des elektrischen Ausgangssignals des Spannungssensors in Zeitabständen, um das für den chirurgischen Eingriff abgegebene Fluidvolumen zu ermitteln,
wobei die eine oder die mehreren Raten die Genauigkeit der Fluidmessung nicht beeinträchtigen und die Fluidmessung nicht auf der Aktivität der Pumpe basiert, sondern auf einem Abgang von Fluid aus dem Behälter und somit einer Gewichtsabnahme des Behälters; und
eine Anzeigevorrichtung (44) zum Anzeigen der während des chirurgischen Eingriffs abgegebenen Fluidmenge.

2. System nach Anspruch 1, wobei der kosmetische chirurgische Eingriff ein aus der folgenden Gruppe ausgewählter Eingriff ist: lipoplastischer Eingriff (Fettabsaugung) und Füllen von Brustimplantaten oder Brustvergrößerem.

3. System nach Anspruch 1, wobei die Anzeigevorrichtung einen Rückstellknopf aufweist, der, wenn gedrückt, die Anzeige auf "Null" stellt.

4. System nach Anspruch 1 oder 2, wobei die Schlauchanordnung aus Polyvinylchlorid hergestellt ist.

5. System nach Anspruch 1 oder 2, wobei die Anzeigevorrichtung die Fluidmenge als Gewicht oder als Volumen anzeigt.

## Revendications

1. Système pour l'administration et le contrôle précis de l'administration d'un volume pertinent d'un fluide stérile à un patient ou à un dispositif implantable lors d'une intervention de chirurgie esthétique, ledit système comprenant :
un capteur à jauge extensométrique (40),
un conteneur (20) de fluide stérile relié au capteur à jauge extensométrique, de telle manière que le capteur à jauge extensométrique génère périodiquement une sortie électrique proportionnelle au poids du fluide et du conteneur ;
une pompe péristaltique (35) destinée à pomper le volume pertinent de fluide stérile du conteneur vers le patient ou le dispositif implantable, ladite pompe péristaltique comportant une commande de vitesse réglable pour administrer le fluide stérile à un ou plusieurs débits compris entre 30 ml/min et 1000 ml/min, le volume pertinent étant compris entre 100 et 5.000 ml ;
une tuyauterie stérile (31, 32) raccordée au conteneur et à la pompe pour administrer le fluide stérile pendant l'intervention chirurgicale ;
un processeur (43) pour le traitement périodique de la sortie électrique de la jauge extensométrique, afin de déterminer le volume de fluide administré pour l'intervention chirurgicale,
le ou les débits n'influençant pas la précision de la mesure de fluide, et la mesure de fluide n'étant pas sensible à l'activité de la pompe, mais à une perte de fluide et une diminution de poids conséquente du conteneur ; et
un écran (44) pour l'affichage de la quantité de fluide administrée pendant l'intervention chirurgicale.

2. Système selon la revendication 1, où l'intervention de chirurgie esthétique fait partie du groupe comprenant la lipoplastie et le remplissage de prothèses mammaires ou de sizers d'implants mammaires.

3. Système selon la revendication 1, où l'écran comporte un bouton reset qui réinitialise l'écran quand il est activé.

4. Système selon la revendication 1 ou la revendication 2, où la tuyauterie est en chlorure de polyvinyle.

5. Système selon la revendication 1 ou la revendication 2, où l'écran affiche la quantité de fluide soit en poids soit en volume.
